# EUROPÄISCHE PATENTSCHRIFT

(11) **EP 2 763 951 B1**
(45) Veröffentlichungstag und Bekanntmachung des Hinweises auf die Patenterteilung: **02.03.2016**
(21) Anmeldenummer: 12766011.6
(22) Anmeldetag: 05.09.2012
(51) Int. Cl.: C07C 53/10, C07C 51/41, C07G 1/00

(54) **VERFAHREN ZUR GEWINNUNG VON ACETAT AUS LIGNOCELLULOSE**
PROCÉDÉ DE RÉCUPÉRATION D'ACÉTATE À PARTIR DE LA LIGNOCELLULOSE
METHOD FOR RECOVERY OF ACETATE FROM LIGNOCELLULOSE

(30) Priorität: 05.10.2011 AT 14432011
(43) Veröffentlichungstag der Anmeldung: 13.08.2014
(73) Patentinhaber: Annikki GmbH, 8020 Graz (AT)
(72) Erfinder: TERS, Thomas, A-1230 Wien (AT); FACKLER, Karin, A-1140 Wien (AT); MESSNER, Kurt, A-1170 Wien (AT); ERTL, Ortwin, A-8076 Vasoldsberg (AT)
(74) Vertreter: Schwarz & Partner
(86) Internationale Anmeldenummer: PCT/EP2012/067314
(87) Internationale Veröffentlichungsnummer: WO 2013/050210

(56) Entgegenhaltungen:
- US-A- 1 695 742
- US-A- 4 395 543
- US-A- 5 264 623

## Beschreibung

Die Erfindung betrifft ein Verfahren zur Gewinnung einer wässerigen Lösung eines Alkaliacetats aus Lignocellulose.

Im Zusammenhang mit der Verknappung von Rohöl gewinnt der nachwachsende Rohstoff Lignocellulose (Stroh, Holz, Papierabfälle etc.) als Ausgangsmaterial für chemische Produkte und Treibstoffe sehr an Bedeutung. Lignocellulose besteht aus den ultrastrukturell vernetzten, polymeren Hauptkomponenten Cellulose, Hemicellulose und Lignin, die häufig etwa 85 - 90% des Rohstoffes ausmachen. Der Rest kann unter dem Begriff niedermolekulare Inhaltstoffe zusammengefasst werden. Unter diesen Inhaltstoffen ist neben den Extraktstoffen und anorganischen Begleitstoffen besonders das Acetat hervorzuheben. Acetylgruppen kommen vor allem in Laubhölzern und Gräsern und anderen Lignocellulosen vor. Meist sind sie in diesen an der Hemicellulose, konkreter im Polymer O-Acetyl-4-O-methylglucurono(arabino)xylan chemisch gebunden. Dem Acetat kommt in mehrfacher Hinsicht große Bedeutung zu. Als Wertstoff kann es in Form von Essigsäure destillativ aus Zellstoffkochlaugen von Laubholzzellstoffen gewonnen werden. Bei der Herstellung von Bioalkohol aus lignocellulosischen Substraten, wie z.B. Stroh ist Acetat ein starker Hemmstoff, der die Alkoholausbeute negativ beeinflusst.

Die Spaltung der als Polymere vorliegenden Komponenten und ihre Auftrennung in einzelne Produktströme sowie deren weitere Verarbeitung zu höherwertigen Produkten ist die Aufgabe von Bioraffinerien der biochemischen Plattform. Die Rentabilität solcher Bioraffinerien ist weitgehend davon abhängig, welche Wertschöpfung aus den Produktströmen erzielt werden kann. Dies wird wiederum massiv von der Reinheit der einzelnen Produktströme beeinflusst, da nachgeschaltete Auftrennungsprozesse schwierig und kostspielig sein können. Als ideal kann also ein Prozess betrachtet werden, bei dem die Spaltung der einzelnen Hauptkomponenten möglichst selektiv erfolgt. Zu diesem Zweck ist es von Vorteil, den Schritt der Extraktstoffextraktion an den Anfang von Bioraffinerieverfahren zu stellen.

Einen starken Einfluss auf die Wertschöpfung des gesamten Prozesses hat neben der Nutzung des Kohlenhydratanteiles die Menge und Qualität des gewonnen Lignins. Lignin gewinnt als Ersatz für petrochemisch erzeugte Aromaten sehr stark an wirtschaftlicher Bedeutung. Die Nutzungsmöglichkeiten des gewonnen Lignins sind wiederum sehr von seiner chemischen Zusammensetzung, vor allem aber vom Molekulargewicht der gewonnenen Ligninfraktion abhängig. Insbesondere schwefelfreie niedermolekulare Ligninfraktionen sind als Rohstoff für Anwendungen in der Kunststoff- und Harzherstellung gefragt. Im Gegensatz zu Kraft- oder Sulfit Zellstofffabriken sind Bioraffinerien darauf ausgerichtet, schwefelfreies Lignin herzustellen.

Unter den, in Bioraffinerien zum Aufschluss von Lignocellulose angewandten Verfahren sind besonders alkalische Verfahren hervorzuheben, deren Aufschlussprinzip primär die Entfernung des Lignins ist. Das zugrundeliegende chemische Prinzip ist die alkalische Esterspaltung, durch die sowohl die Verbindung zwischen Lignin und Hemicellulose, als auch die Essigsäure-Hemicellulose Ester gespalten werden. Ein derartiges Verfahren ist in US 4,395,543, Avgerinos und Wang (1981) beschrieben. Es wird ein Verfahren zum Aufschluss von Lignocellulose beschrieben, bei dem eine Extraktionslösung bestehend aus Wasser, zwischen 40 und 75% Alkohol und einem pH zwischen 11 und 14 verwendet wird. Zusätzlich geht aus dem Patent hervor, dass bei Erhöhung der Ethanolkonzentration bis auf 100% die Menge des freigesetzten Lignins gegen Null geht. Weiters wird beschrieben, dass mit Erhöhung der Alkoholkonzentration auf 100% auch die Menge der freigesetzten Zucker gegen Null geht. Der Verlauf der Acetatfreisetzung wird nicht beschrieben.

In der US 1,695,742 ist die Gewinnung von Natriumacetat aus Holz durch Behandlung des Holzes mit Natriumsulfit und Natrumbicarbonat, Zugabe eines calciumhältigen, anorganischen Materials (lime) und NaOH zur erhaltenen Lösung und Erhitzen und Extraktion des entstandenen Natriumacetats mit Wasser beschrieben.

US 5,264,623 offenbart ein Verfahren zur Gewinnung von Calciumsalzen aus Cellulose, Hemicellulose oder Stärke enthaltender Biomasse, durch Pyrolyse der Biomasse, Destillieren des erhaltenen Produkts, wobei ein Destillat erhalten wird, das zumindest eine Säure ausgewählt aus Essigsäure, Ameisensäure, Propionsäure und deren Ester, oder Formaldehyd enthält, Zugabe einer alkalischen Calciumquelle zum Destillat zur Salzbildung und Abdestillieren von Wasser und flüchtigen Bestandteilen zum Erhalt von Calciumacetat, Calciumformat, Calciumpropionat in fester Form.

In der vorliegenden Patentanmeldung wird gezeigt, dass bei Auswahl bestimmter Reaktionsbedingungen, in erster Linie der Auswahl der richtigen Alkoholkonzentration in einer wässrigen alkalischen Lösung, Acetat überraschenderweise annähernd quantitativ aus Stroh gewonnen werden kann.

Die vorliegende Erfindung stellt ein Verfahren zur Gewinnung einer wässerigen Lösung eines Alkaliacetats aus Lignocellulose zur Verfügung, wobei Lignocellulose mit einer ersten wässerigen Extraktionslösung mit einem pH-Wert von 12 bis 14 und einem Gehalt an einem C₁₋₄-Alkohol, insbesondere Ethanol oder Isopropanol, von 40% bis 90%, insbesondere 75% bis 85%, behandelt wird, wobei eine erste wässerige Lösung eines Alkaliacetats erhalten wird, und wobei diese erste Lösung eines Alkaliacetats dazu verwendet wird, weitere Lignocellulose zu behandeln, um weiteres Acetat in der ersten Lösung anzureichern.

Ein Verfahren, das durch die vorliegende Erfindung zur Verfügung gestellt wird, wird hier auch als "Verfahren gemäß (nach) vorliegender Erfindung" bezeichnet.

In einem Verfahren gemäß vorliegender Erfindung haben sich als Lignocellulose insbesondere Laubholz, Stroh, Bagasse oder ein- und mehljährige Gräser als vorteilhaft erwiesen.

Ein Vorteil eines Verfahrens gemäß vorliegender Erfindung ist die derart erreichbare hohe Acetatkonzentration und dadurch leichtere Abtrennung des Acetats.

Ein weiterer Vorteil ist die damit verbundene Verminderung des Bedarfs an Acetat-Extraktionslösung.

Ein weiterer Vorteil ist die weitgehende Trennung von Acetat und Lignin, die ansonsten in einer Lösung gemeinsam anfallen würden.

Ein weiterer Vorteil ist der Umstand, dass man für die Rezyklierung der LIGNIN-Lösung weniger NaOH zuzusetzen braucht, da weniger Acetat in Lösung ist.

Ein weiterer Vorteil ist der Umstand, dass durch den verminderten Bedarf an Lauge (NaOH) weniger Salze anfallen.

Ein weiterer Vorteil liegt darin, dass dadurch die LIGNIN-Lösung auf neue Mengen Stroh eingesetzt werden kann und dadurch auch die Konzentration an Lignin in Lösung gesteigert werden kann, bzw. die in Relation zum Stroh benötigte Menge an Lösemitteln reduziert werden kann.

Ein weiterer Vorteil liegt darin, dass weitere Lignin-Extraktionsschritte nicht durch das Vorhandensein von Acetat gestört werden.

Ein weiterer Vorteil liegt darin, dass nach einem weiteren Lignin-Extraktionsschritt Acetat nicht gesondert abgetrennt werden muss.

In einem Verfahren gemäß vorliegender Erfindung kann überraschenderweise bei einer Temperatur von 70°C und einem pH-Wert von 12 bis 14 bei einem Alkoholgehalt von 40% bis 90% bereits nach 30 Minuten Acetat in hoher Ausbeute aus Weizenstroh extrahiert werden. Wird der Alkoholgehalt bei diesen Bedingungen über 85% erhöht, so sinkt die Menge des extrahierten Acetats ab.

Es wurde gefunden, dass eine Ethanolkonzentration von 80% in einem Verfahren gemäß vorliegender Erfindung als optimal für die Extraktion von Acetat aus Stroh zu betrachten ist, da bei 80% Ethanol auch die Menge der freigesetzten Zucker sehr niedrig ist und eine hohe Selektivität der Acetatgewinnung erreicht werden kann.

Weiters hat sich gezeigt, dass bei den oben erwähnten Bedingungen, bei 80% Ethanol bereits nach 30 Minuten 16% des Lignins freigesetzt wird. Überraschenderweise hat sich herausgestellt, dass das gewonnene Lignin ein sehr niedriges Molekulargewicht (Mw 1340, Mn 850) mit sehr schmaler Molekulargewichtsverteilung (Pd 1,58) aufweist.

In einem Verfahren gemäß vorliegender Erfindung wird also gezeigt, dass überraschenderweise bei den beschriebenen optimalen Bedingungen gleichzeitig Acetat quantitativ und 16% sehr niedermolekulares Lignin aus Stroh gewonnen werden können.

Weiters wurde gemäß vorliegender Erfindung gefunden, dass die extrahierten Komponenten Acetat und Lignin durch mehrmaliges Rezyklieren der Extraktionslösung auf jeweils frisches Lignocellulosesubstrat unter jeweiliger vorheriger Zudosierung der verbrauchten Lauge, z. B. Natronlauge, aufkonzentriert werden können. Wie im Beispiel 4 dargestellt, nimmt mit den Rezyklierungsschritten überraschenderweise sowohl die Menge des Acetats, wie auch des Lignins linear in der Rezyklierungslösung zu und folgt nicht einer Sättigungskmve, wie zu erwarten gewesen wäre. Nach 7 Zyklen konnte der Acetatgehalt von 0,94 mg/ml auf 6,42 mg/ml und der Ligningehalt von 1,88 mg/mL auf 13,67 mg/ml erhöht werden. Die Anzahl der Extraktionszyklen kann je nach gewünschter Endkonzentration der Extraktstoffe frei gewählt werden, oder z.B. bis zur Sättigung durchgeführt werden.

Wie aus Beispiel 5 ersichtlich ist, kann die Extraktion bei längerer Reaktionszeit auch bei Raumtemperatur durchgeführt werden und führt zu ähnlichen Ergebnissen wie bei 70°C (siehe Beispiel 4).

Durch die erfolgreiche Aufkonzentrierung wird eine Endkonzentration erreicht, die die Abtrennung des Acetats und des niedermolekularen Lignins erst wirtschaftlich vertretbar macht. Weiters wird durch die Rezyklierung die Menge des zu verwendenden Alkohols bezogen auf die gesamte behandelte Menge Biomasse drastisch gesenkt.

### Beschreibung der Abbildungen

Fig. 1 zeigt den zeitlichen Verlauf (in Minuten) der Acetatkonzentration (mg/mL) in der Extraktionslösung bei 70°C und unterschiedlichen Ethanolgehalten (EtOH), wobei (1) 40% EtOH, (2) 60% EtOH, (3) 80% EtOH, (4) 90% EtOH, (5) 95% EtOH und (6) 100% EtOH in der Extraktionslösung bezeichnet.
Fig. 2 zeigt die Zunahme des Acetatgehaltes (mg/mL) in der Extraktionslösung bei Recyklierung der Lösung. Der Anstieg über 7 Zyklen ist praktisch linear.
Fig. 3 zeigt die Zunahme des Ligningehaltes (mg/mL) in der Extraktionslösung bei Recyklierung der Lösung. Der Anstieg über 7 Zyklen ist praktisch linear.
Fig. 4 zeigt den Acetatgehalt (a) und den Ligningehalt (b) in mg/mL nach der jeweiligen Rezyklierung der Extraktionslösung bei Raumtemperatur (25°C) in den Zyklen 1 bis 4.

Mit den nachfolgenden Beispielen werden bevorzugte Ausführungsformen der Erfindung noch näher beschrieben.

### Beispiel 1

### Zeitlicher Verlauf der Acetatkonzentration in der Extraktionslösung bei 70°C und unterschiedlichen Ethanolgehalten

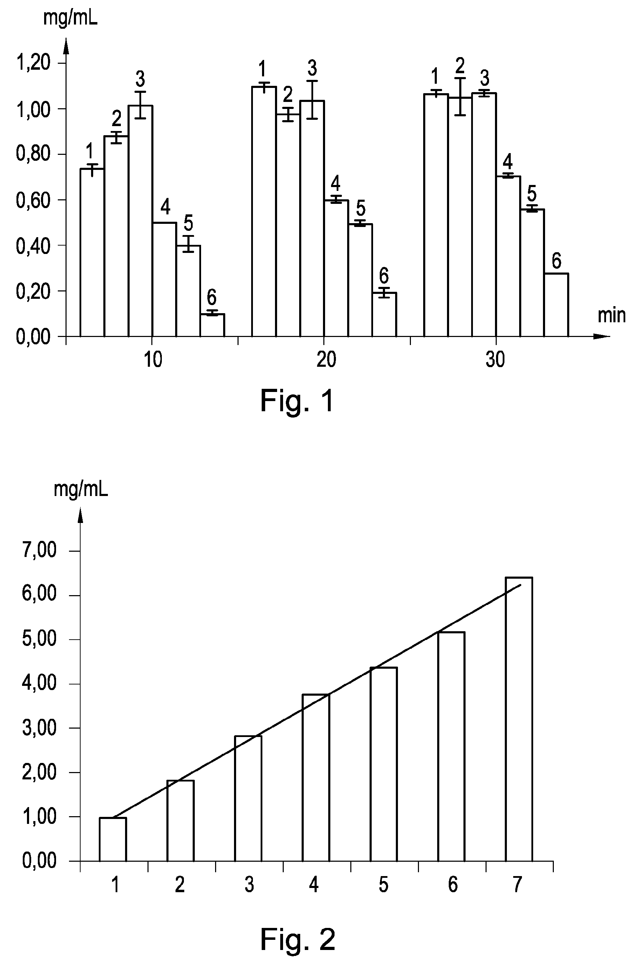

10 g zerkleinertes Weizenstroh wurden in einem 500 mL Reaktionsgefäß in 200 mL (5% Feststoffanteil) einer auf 70°C vortemperierten Lösung, bestehend aus Wasser/Ethanol in unterschiedlichen Verhältnissen (40%, 60%, 80%, 90%, 95%, 100% EtOH) und 0,8g NaOH suspendiert. Die Suspension wurde 10, 20 oder 30 Minuten bei 200 rpm und 70°C kontinuierlich magnetisch gerührt. Danach wurde der Feststoffanteil durch Filtration abgetrennt und der Acetatgehalt der Lösung enzymatisch bestimmt. Wie in Fig. 1 zu erkennen ist werden bei Ethanolkonzentrationen zwischen 60% und 80% in dem untersuchten Zeitraum die meisten Acetylgruppen hydrolysiert.

### Beispiel 2

### Mengen des extrahierten Lignins bei unterschiedlichen Ethanolgehalten

10 g zerkleinertes Weizenstroh wurden in einem 500 mL Reaktionsgefäß in 200 mL (5% Feststoffanteil) einer auf 70°C vortemperierten Lösung, bestehend aus Wasser/Ethanol in unterschiedlichen Verhältnissen (40%, 60%, 80%, 90%, 95%, 100% EtOH) und 0,8g NaOH suspendiert. Die Suspension wurde bei 200 rpm und 70°C für 24 Stunden kontinuierlich magnetisch gerührt. Danach wurde der Feststoffanteil durch Filtration abgetrennt. Der Ligningehalt in der Lösung wurde photometrisch bei 280 nm bestimmt.

**Tabelle1: Mengen des extrahierten Lignins bei unterschiedlichen Ethanolgehalten**

| | |
|---|---|
| 40% Ethanol | 80% |
| 60% Ethanol | 75% |
| 80% Ethanol | 25% |
| 100% Ethanol | 5% |

### Beispiel 3

### Bestimmung des Molekulargewichtes einer bei 80% Ethanol gewonnen Ligninlösung

10 g zerkleinertes Weizenstroh wurden in einem 500 mL Reaktionsgefäß in 200 mL (5% Feststoffanteil) einer auf 70°C vortemperierten Lösung, bestehend aus 20% Wasser, 80% Ethanol und 0,8 g NaOH suspendiert. Die Suspension wurde bei 200 rpm und 70°C für 30 Minuten kontinuierlich magnetisch gerührt. Danach wurde der Feststoffanteil durch Filtration abgetrennt.

Das mittlere Molekulargewicht des bei 80% Ethanol extrahierten Lignins wurde mittels eines alkalischen HPSEC Systems (Tosoh Bioscience TSK PW 5000-3000) untersucht.

**Tabelle 2 (M_{w} weight average, Mₙ number average, P_{d} Polydisperistät):**

| | |
|---|---|
| M_{w} | 1340 |
| Mₙ | 850 |
| P_{d} | 1,58 |

### Beispiel 4

### Recyklierung zur Acetat- bzw. Ligningewinnung Extraktionsstufe

10 g zerkleinertes Weizenstroh wurden in einem 500 mL Reaktionsgefäß in 200 mL (5% Feststoffanteil) einer Lösung, bestehend aus 20% Wasser, 80% Ethanol und 0,8 g NaOH suspendiert. Die Suspension wurde 30 Minuten bei 200 rpm und 70°C kontinuierlich magnetisch gerührt. Nach der Extraktion wurde die Lösung durch Filtration vom Feststoff abgetrennt, mit neuer NaOH auf den Ausgangs pH-Wert gestellt und frisches Stroh (5% w/v) zugesetzt.

Die Suspension wurde wieder unter den oben beschriebenen Bedingungen behandelt, und nach der Abtrennung des Feststoffes einem weiteren Recyklierungsschritt unterzogen.

Vor jedem Recyklierungschritt wurde eine Probe gezogen und der Ligningehalt sowie der Acetatgehalt der Lösung bestimmt.

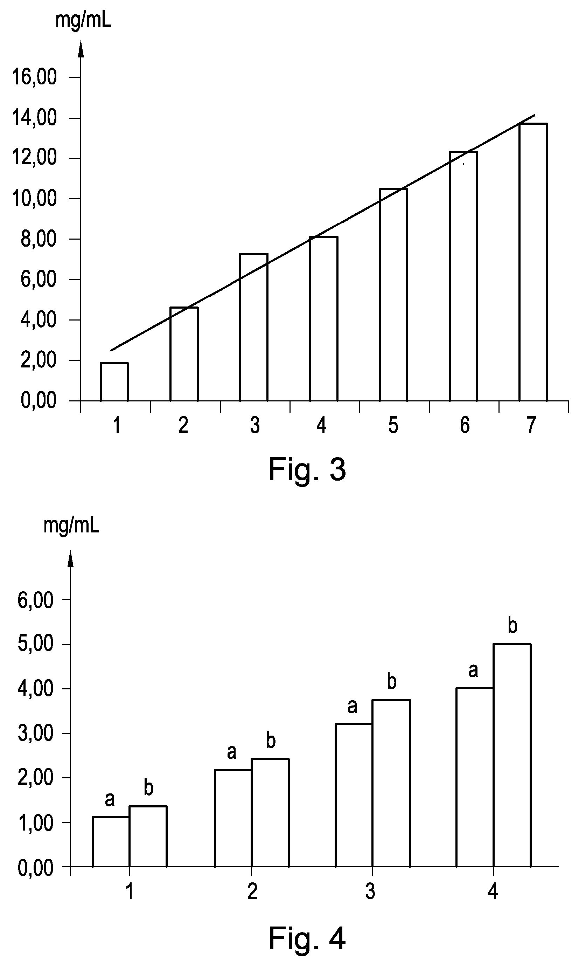

Wie in den Fig. 2 und Fig. 3 zu erkennen ist, steigen sowohl Ligninkonzentrationen als auch die Acetatkonzentration in der Lösung mit jedem Recyklierungs-Schritt linear an.

Aus dem jeweils frischen Feststoff wurden im Durchschnitt 0.91 mg/mL Acetat und 1.97 mg/mL Lignin pro Extraktionsschritt entfernt. Abweichungen von diesen Werten können durch die Variabilität des Extraktionsgutes erklärt werden.

### Beispiel 5

### Zeitlicher Verlauf der Acetatkonzentration in der Extraktionslösung bei 25°C

10 g zerkleinertes Weizenstroh wurden in einem 500 mL Reaktionsgefäß in 200 mL (5% Feststoffanteil) einer Lösung, bestehend aus 20% Wasser, 80% Ethanol und 0,8 g NaOH suspendiert. Die Suspension wurde 16 Stunden bei 200 rpm bei 25° C kontinuierlich magnetisch gerührt. Nach der Extraktion wurde die Lösung durch Filtration vom Feststoff abgetrennt, mit neuer NaOH auf den Ausgangs pH-Wert gestellt und frisches Stroh (5% w/v) zugesetzt.

Die Suspension wurde wieder unter den oben beschriebenen Bedingungen behandelt, und nach der Abtrennung des Feststoffes einem weiteren Recyklierungsschritt unterzogen. Vor jedem Recyklierungschritt wurde eine Probe gezogen und der Ligningehalt sowie der Acetatgehalt der Lösung bestimmt. Die (linearen) Zunahmen des Acetat- und Ligningehalts in der Extraktionslösung bei Rezyklierung der Lösung in 4 Zyklen ist in Fig. 4 gezeigt.

## Patentansprüche

1. Verfahren zur Gewinnung einer wässerigen Lösung eines Alkaliacetats aus Lignocellulose, wobei Lignocellulose mit einer ersten wässerigen Extraktionslösung mit einem pH-Wert von 12 bis 14 und einem Gehalt an einem C₁₋₄-Alkohol von 40% bis 90% behandelt wird, wobei eine erste wässerige Lösung eines Alkaliacetats erhalten wird, und wobei diese erste Lösung eines Alkaliacetats dazu verwendet wird, weitere Lignocellulose zu behandeln, um weiteres Acetat in der ersten Lösung anzureichern.

2. Verfahren nach Anspruch 1, worin der C₁₋₄-Alkohol Ethanol ist.

3. Verfahren nach Anspruch 1, worin der C₁₋₄-Alkohol Isopropanol ist.

4. Verfahren nach einem der Ansprüche 1 bis 3, worin der Gehalt an einem C₁₋₄-Alkohol von 75% bis 85% beträgt.

5. Verfahren nach einem der Ansprüche 1 bis 4, **dadurch gekennzeichnet, dass** als Lignocellulose Laubholz, Stroh, Bagasse oder ein- und/oder mehrjährige Gräser eingesetzt werden.

## Claims

1. A method of obtaining an aqueous solution of an alkali acetate from lignocellulose, wherein lignocellulose is treated with a first aqueous extraction solution having a pH value in the range of 12 to 14 and a content of a C₁₋₄ alcohol in the range of 40% to 90%, whereby a first aqueous solution of an alkali acetate is obtained, and wherein said first solution of an alkali acetate is used to treat additional lignocellulose in order to enrich additional acetate in said first solution.

2. A method according to claim 1, wherein the C₁₋₄ alcohol is ethanol.

3. A method according to claim 1, wherein the C₁₋₄ alcohol is isopropanol.

4. A method according to any of claims 1 to 3, wherein the content of a C₁₋₄ alcohol is in the range of 75% to 85%.

5. A method according to any of claims 1 to 4, **characterized in that** as lignocellulose hardwood, straw, bagasse or annual and/or perennial grasses are used.

## Revendications

1. Procédé servant à récupérer une solution aqueuse d'un acétate alcalin à partir de lignocellulose, sachant que la lignocellulose est traitée à l'aide d'une première solution d'extraction aqueuse présentant une valeur pH allant de 12 à 14 et une teneur en un alcool en C₁-C₄ allant de 40 % à 90 %, sachant qu'une première solution aqueuse d'un acétate alcalin est obtenue, et sachant que ladite première solution d'un acétate alcalin est utilisée afin de traiter une autre lignocellulose afin d'enrichir un autre acétate dans la première solution.

2. Procédé selon la revendication 1, dans lequel l'alcool en C₁-C₄ est de l'éthanol.

3. Procédé selon la revendication 1, dans lequel l'alcool en C₁-C₄ est de l'isopropanol.

4. Procédé selon l'une quelconque des revendications 1 à 3, dans lequel la teneur en un alcool en C₁-C₄ présente une valeur allant de 75 % à 85 %.

5. Procédé selon l'une quelconque des revendications 1 à 4, **caractérisé en ce que** sont employés en tant que lignocellulose du bois de feuillus, de la paille, de la bagasse ou des graminées annuelles et/ou vivaces.
